# EUROPÄISCHE PATENTSCHRIFT

(11) **EP 1 194 145 B1**
(45) Veröffentlichungstag und Bekanntmachung des Hinweises auf die Patenterteilung: **05.02.2003**
(21) Anmeldenummer: 00947853.8
(22) Anmeldetag: 17.06.2000
(51) Int. Cl.: A61K 31/4174, A61K 31/415, A61K 9/08, A61K 9/00

(54) **STABILE XYLOMETAZOLIN- UND OXYMETAZOLINLÖSUNG**
STABLE XYLOMETAZOLINE AND OXYMETAZOLINE SOLUTION
SOLUTION DE XYLOMETAZOLINE ET D'OXYMETAZOLINE STABLE

(30) Priorität: 22.06.1999 US 337789
(43) Veröffentlichungstag der Anmeldung: 10.04.2002
(73) Patentinhaber: BOEHRINGER INGELHEIM INTERNATIONAL GMBH, 55216 Ingelheim am Rhein (DE)
(72) Erfinder: MAERZ, Frieder, Ulrich, D-55270 Soergenloch (DE)
(74) Vertreter: Laudien, Dieter, Dr.
(86) Internationale Anmeldenummer: EP0005583
(87) Internationale Veröffentlichungsnummer: WO00078297

(56) Entgegenhaltungen:
- WO-A-99/38492
- US-A- 4 603 131
- US-A- 4 970 240
- US-A- 5 801 199

## Beschreibung

Die vorliegende Erfindung betrifft eine biologisch und chemisch stabile Xylometazolinund/oder Oxymetazolinlösung mit Glycerol und/oder Sorbitol als Adjuvans.

### Hintergrund der Erfindung

Xylometazolin [2- (4-tert.Butyl-2,6-dimethylbenzyl)-4,5-dihydro-1-*H*-imidazol] ist, wie Oxymetazolin [6-tert-Butyl-3-(4,5-dihydro-1-*H*-imidzol-2-ylmethyl)-2,4-dimethylphenol], ein Vasokonstriktor aus der Klasse der Imidazol-Wirkstoffe. Beide können als Rhinologikum verwendet werden. Bei Verwendung als Rhinologikum werden diese Substanzen in Form einer wässrigen Lösung mit Hilfe einer Nasenspray-Pumpe appliziert. Da bekannt ist, daß die freien Basen Xylometazolin und Oxymetazolin für pharmazeutische Zwecke in wässriger Lösung nur wenig hydrolysestabil sind, wird der Wirkstoff in der Regel nur in Form eines Salzes, insbesondere des Hydrochlorids eingesetzt.

Sprayfähige Rhinologika mit Xylometazolin bzw. Oxymetazolin, in denen der Wirkstoff nicht als Hydrochlorid vorliegt, offenbart die WO 88/00473. Gemäß dieser Patentschrift kann Xylometazolin als freie Base wasserfrei zusammen mit einem ätherischen Öl in einem Triglycerid ohne weiteren Stabilisator formuliert werden.

Rhinologischen Lösungen mit Xylometazolin- und Oxymetazolinhydrochlorid werden Konservierungsmittel beigemischt. Diese verhindern eine Kontamination mit Bakterien und anderen Mikroorganismen während der Lagerung und Benutzung der Lösung. Konservierungsmittel sind insbesondere dann notwendig, wenn diese Formulierungen weitere Bestandteile enthalten, die ein Wachstum von Mikroorganismen fördern. Solche Bestandteile können beispielsweise Puffer auf Basis von Zitronensäure, Milchsäure, Propionsäure usw., Adjuvantien oder andere Verbindungen sein. Bei den Adjuvantien, die in Formulierungen mit Xylometazolin bzw. Oxymetazolin eingesetzt werden, handelt es sich in der Regel um Polyvinylpyrrolidon, Polysorbate, verschiedene Cellulosederivate und/oder Polyalkohole, wie Glycerol und Sorbitol. Besonders von wäßrigen Lösungen mit niedrig dosiertem Sorbitol und/oder Glycerol ist bekannt, daß sie einen sehr guten Nährboden für Mikroorganismen bilden [*M. Barr, L.F. Tice, Journal of the American Pharmaceutical Association, Scientific Edition, 46 (4), 1957, 217-218*]. Daher müssen insbesondere pharmazeutischen Lösungen, die Glycerol oder Sorbitol enthalten, Konservierungsstoffe zugesetzt werden [*M. Barr, L.F. Tice, Journal of the American Pharmaceutical Association, Scientific Edition, 46 (4), 1957, 221*-222]. Zu den von den Autoren untersuchten Konservierungsmitteln gehören Natriumbenzoat, Benzoesäure, Methylparaben, Ethylparaben, Propylparaben, Butylparaben, Cetylpyridiniumchlorid, Benzethoniumchlorid, Natriumdehydroacetat, Saligenin, Sorbinsäure, Benzalkoniumchlorid u.a..

In diesem Zusammenhang ist erwähnenswert, daß in der Literatur für wäßrige Lösungen mit einem hohen Anteil an Glycerol und Sorbitol ein Umschlagen des, das Wachstum von Mikroorganismen begünstigenden, Effekts in das Gegenteil diskutiert wird *[H.P.Fiedler, Lexikon der Hilfsstoffe für Pharmazie, Kosmetik und angrenzende Gebiete, Editio Cantor Verlag, 4. Auflage*, *S. 1424]*. Nach M. Barr und L.F.Tice tritt dieser inhibitive Effekt von höher konzentrierten Glycerol- bzw. Sorbitol-Lösungen u.a. in Abhängigkeit des pH-Werts der Lösung und der biologischen Spezies auf. So beobachten die Autoren, daß der inhibitive Effekt von Glycerol für die empfindlichste Spezies Pseudomonas aeruginosa bei einem pH-Wert von 7,4 erst ab 30 Gew.% auftritt *[M. Barr, L.F. Tice, Journal of the American Pharmaceutical Association, Scientific Edition, 46 (4), 1957, 217-218]*, bei einem mit HCl und NaOH eingestelltem pH-Wert von 5,6 ab 25 Gew.% *[M. Barr, L.F. Tice, Journal of the American Pharmaceutical Association, Scientific Edition, 46 (4), 1957, 219-221].* Für Sorbitol sind die Werte im Neutralen identisch, unter den sauren Bedingungen dagegen werden 40 Gew.% benötigt, um den inhibitiven Effekt auf P. aeruginosa auszulösen. *[M. Barr, L.F. Tice, Journal of the American Pharmaceutical Association, Scientific Edition, 46 (4), 1957, 221-222].* Diese Beobachtungen für Glycerol werden von anderen Autoren in ähnlicher Weise unterstützt *[E. Mariani, C.J. Libbey*, *W. Litsky, Development in industrial Microbiology 14 1973, 356-360].* In rhinologischen Lösungen liegt die Menge an Sorbitol bzw. Glycerol stets unterhalb dieser antimikrobiell wirksamen Menge, also in dem Bereich, der das Wachstum von Mikroorganismen fördern kann.

Konservierungsmittel haben jedoch verschiedene Nachteile, insbesondere in Rhinologika. Sie können nicht nur die Abwehrmechanismen der Nasenschleimhaut, die Phagozytose, Chemotaxis und das mukoziliäre Transportsystem schädigen, sondern auch Zellschädigungen, allergische Reaktionen und sonstige Reizungen verursachen. Auf der anderen Seite muß bei Formulierungen, bei denen Konservierungsmittel weggelassen werden, mit erheblicher mikrobiologischer Kontamination während der Lagerung oder Benutzung gerechnet werden, insbesondere wenn die Formulierung weitere Bestandteile enthält, die das Wachstum von Mikroorganismen fördern.

### Beschreibung der Erfindung

Die Aufgabe der vorliegenden Erfindung besteht darin, eine isotonische Lösungsformulierung mit einem Imidazol-Wirkstoff zu schaffen, die die aus dem Stand der Technik bekannten Nachteile überwindet.

Es ist auch eine Aufgabe der Erfindung, eine isotonische Lösung mit einem Imidazol-Wirkstoff als Rhinologikum zu formulieren, die nur einen minimalen Anteil an weiteren Zusatzstoffen enthält, um eine Reizung der Nasenschleimhaut maximal zu reduzieren.

Es ist eine weitere Aufgabe der vorliegenden Erfindung, ein Rhinologikum mit einem Imidazol-Wirkstoff und einem Polyalkohol als Adjuvans zu formulieren, wobei die resultierende Lösung keine oder nahezu keine weiteren Substanzen enthält, die das Wachstum von Mikroorganismen fördert.

An dieser Stelle sei angemerkt, daß im Kontext mit dieser Erfindung nicht zwischen "bakteriostatisch und bakterizid" u.ä. unterschieden wird. Statt dessen werden diese Effekte unter Begriffen wie "keinen geeigneten Nährboden für Mikroorganismen", "ein das Wachstum von Mikroorganismen negativ beeinflussende Effekt" oder "antibakterielle Wirkung/Effekt", "keine Anfälligkeit gegenüber mikrobieller Kontamination" etc. ohne nähere Differenzierung subsumiert.

Überraschend wurde nun gefunden, daß mit bestimmten Puffern neutrale bis schwach sauer gepufferte isotonische Lösungen mit einem oder beiden der beiden Imidazol-Wirkstoffe Xylometazolin- und/oder Oxymetazolinhydrochlorid, die Sorbitol und/oder Glycerol mit einem Anteil von weniger als 10 Gew.% enthalten, keinen geeigneten Nährboden für Mikroorganismen bilden, sondern das Wachstum von Mikroorganismen sogar negativ beeinflussen.

Die vorliegende Erfindung löst die ihr gestellte Aufgabe dadurch, daß eine stabile Lösungsformulierung mit Xylometazolin und/oder Oxymetazolin als Wirkstoff geschaffen wird, die den Wirkstoff, ein für die nasale Applikation pharmazeutisch akzeptables Lösungsmittel wie Wasser, ein Adjuvans aus der Gruppe Sorbitol und/oder Glycerol und den besagten pH-Puffer enthält.

Die Formulierung ist isotonisch eingestellt.

Ein Vorteil der erfindungsgemäßen Formulierung besteht darin, daß auf die Verwendung von herkömmlichen Konservierungsstofffen, wie beispielsweise Benzalkoniumchlorid, Chlorhexidin Gluconat, Benzylalkohol, Dinatriumethylendiamintetraacetat oder Thimerisol verzichtet werden kann.

Dabei ist entscheidend, daß die Formulierung dennoch derart ist, daß sie keine Kontamination mit Mikroorganismen fördert, die zu einer Anhäufung von Mikroorganismen der Formulierung während der Lagerzeit oder Applikationszeit über ein pharmazeutisch vertretbares Maß führt.

Dadurch, daß in der Formulierung auf die besagten Konservierungsmittel verzichtet werden kann, werden die aus dem Stand der Technik mit der Verwendung von Konservierungsmitteln in rhinologischen Formulierungen bekannten Schwierigkeiten überwunden.

Als Puffer für die erfindungsgemäße Formulierung eignen sich pharmazeutisch akzeptable anorganische Puffer oder der organische Puffer Trometamol. Bevorzugt sind Puffer auf Basis von anorganischen Alkali-Phosphaten und Alkaliboraten, bevorzugt die entsprechenden Natrium- und/oder Kalium-Salze. Ganz besonders bevorzugt sind Puffer auf Basis von Mononatriumdihydrogen-Dinatriummonohydrogen-Phosphat und/oder die analogen Kalium-Salze.
Als organischer Puffer ist Trometamol bevorzugt.

Über das Puffersystem wird ein pH-Wert von 4,0 bis 7,5 eingestellt. Bevorzugt ist ein pH-Wert von 5,0-7,2. Gegebenenfalls kann der pH-Wert durch weitere Zugabe von Salzsäure und/oder Natronlauge korrigiert werden.
Für anoganische Puffer ist ein pH-Wert von 5,5-6,8, ganz besonders von 5,8 bis 6,0 bevorzugt.
Für Trometamol ist ein pH-Wert von 6,1-6,3 bevorzugt.

Im Fall des Trometamols kann eine Menge von 0,2 bis 0,6 Gew.% bezogen auf die Formulierung eingesetzt werden, bevorzugt eine Menge von 0,25 bis 0,45 Gew.% und ganz besonders bevorzugt eine Menge von 0,39 Gew.%.

Isotonische Lösungen mit Xylometazolin und/oder Oxymetazolin als Wirkstoff, Sorbitol und/oder Glycerol, den zum Einstellen der Isotonie notwendigen Substanzen und einem anorganischen Puffer oder Trometamol, die ansonsten keinen weiteren Zusatzstoffe beinhalten und dennoch gegen Kontamination durch Mikroorganismen in einem pharmazeutisch nicht mehr vertretbaren Maß gefeit sind, sind nicht bekannt.

Die Konzentration des Xylometazolin und/oder Oxymetazolin, bzw. deren Hydrochloride, liegt für jeden dieser Wirkstoffe in dem für ihn eigenen für die nasale Applikation geeigneten Bereich, bevorzugt in einer Konzentration zwischen 0,01 und 1,0 Gew.%, besonders bevorzugt zwischen 0,01 und 0,5 Gew.% und ganz besonders bevorzugt zwischen 0,05 und 0,1 Gew.%.

Als Lösungsmittel können alle, für die nasale Applikation pharmazeutisch akzeptablen Lösungsmittel, wie Wasser oder ein Ethanol-Wasser-Gemisch eingesetzt werden. Bevorzugtes Lösungsmittel ist Wasser.

Als Adjuvans kann Sorbitol, Glycerol oder ein Gemisch aus beidem eingesetzt werden. Bevorzugt wird entweder Sorbitol oder Glycerol verwendet. Die Aufgabe dieses Adjuvans liegt zum einen darin, die Löslichkeit des Wirkstoffs in dem Lösungsmittel zu verbessern und zum anderen darin, als Feuchthaltemittel ein Austrocknen der Nasenschleimhaut zu vermeiden.

In einer Ausführungsform der Erfindung beträgt der Anteil des Adjuvans 1 bis 10 Gew.%, bevorzugt 2 bis 6 Gew.%. Für Sorbitol beträgt der Anteil besonders bevorzugt 3,5 - 4,5 Gew.%, ganz besonderes bevorzugt 4,0 Gew.%, für Glycerol beträgt der Anteil bevorzugt 2,0 bis 2,8 Gew.%, ganz besonders bevorzugt 2,4 Gew.%.

Es wurde auch gefunden, daß der das Wachstum von Mikroorganismen negativ beeinflussende Effekt verstärkt wird, wenn die Formulierung mit Hilfe einer Spray- oder Inhalationsvorrichtung appliziert wird, die zwischen Wirkstoffreservoir und Sprühkopf Bestandteile aus oligodynamisch wirksamen Metallen, wie beispielsweise Silber aufweist. Eine solche Sprayvorrichtung ist beispielsweise durch die WO 97/18902 offenbart, auf die hiermit ausdrücklich Bezug genommen wird. Unter oligodynamischen Substanzen werden Metalle oder Metallionen mit keimtötender Wirkung verstanden. Dazu zählen beispielsweise Silber oder Kupfer.

Interessanterweise tritt in diesen Fällen der verstärkende antimikrobielle Effekt sowohl dann auf, wenn die oligodynamisch wirkende Substanz nach einiger Zeit in der Formulierung nachgewiesen werden kann, als auch dann, wenn die oligodynamische Substanz auch nach Lagerung und Gebrauch in einer solchen Sprayvorrichtung nicht nachgewiesen werden kann.

Daher betrifft die Erfindung auch solche Xylometazolin und/oder Oxymetazolin-haltigen Lösungen der oben beschriebenen Art, die zusätzlich eine oligodynamisch wirkende Substanz wie Silber in pharmazeutisch akzeptablen Mengen beinhalten. Derartige Formulierungen sind nicht bekannt.

Die Formulierung enthält neben dem Wirkstoff, den oben beschriebenen Adjuvantien und einem Puffer der oben beschreibenen Art keine weiteren organischen Zusatzstoffe, insbesondere nicht solche wie beispielsweise Zitronensäure, andere organische Säuren oder deren Salze.

Die beschriebene Formulierung eignet sich zur Verwendung als Rhinologikum.

### Beispiele

Im Folgenden soll die Erfindung anhand einiger Untersuchungen zur biologischen Stabilität näher erläutert werden.

Untersuchungen zur biologischen Stabilität werden in Anlehnung an die Prüfung auf ausreichende Konservierung (EuAB 1997, 5.1.3) durchgeführt. Dabei werden 990 µl jeder der zu untersuchenden Formulierung gemäß den Vorschriften des EuAB 1997 mit 10 µl einer Keimlösung, die einer Menge von ca. 10⁵ bis 10⁶ koloniebildenden Einheiten (KBE bzw. CFU) pro ml entspricht, beimpft. Die so erhaltene Lösung wird 14 Tage bei Zimmertemperatur gelagert und über den gesamten Zeitraum wird zu bestimmten Zeiten die Keimzahlveränderung bestimmt.

Die Impfkeimlösung wird aus 18 bis 24 Stunden (bei Bakterien) bzw. einige Tage (bei Pilzen) alten Kulturen in physiologischer Kochsalzlösung gewonnen.

Als Testorganismen werden E. coli ATCC 8739, Ps. aeruginosa ATCC 9027, St. aureus ATCC 6538P eingesetzt.

Die Keimzahlbestimmung erfolgt, indem zu den Zeitpunkten t = 0 h, 6 h, 24 h, 7d und 14d jeweils 50 µl Probe entnommen werden. Daraus wird eine Verdünnungsreihe in physiologischer Kochsalzlösung hergestellt. Die Verdünnungen werden auf Agarplatten aufgetragen, so daß nach geeigneter Inkubation die Vitalkeimzahl ermittelt werden kann.

Für jede der zu untersuchenden Formulierungen wird parallel eine zweite Untersuchung durchgeführt, die von der oben beschriebenen dadurch abweicht, daß in die mit den Keimen geimpfte Testlösung (1 ml) ein Silberfaden eingetaucht wird.

### Beispiel 1:

Untersuchung der folgenden 0,05 Gew.%igen Xylometazolinlösung mit einem pH-Wert von 6,0 auf biologische Stabilität:

| mg / 10 ml = 10150 mg | | |
|---|---|---|
| (01) | Xylometazolin Hydrochlorid | 5,0 |
| (02) | Sorbitol | 400,0 |
| (03) | Mononatriumdihydrogenphophat-Dihydrat | 40,0 |
| (04) | Dinatriummonohydrogenphophat-Dihydrat | 6,5 |
| (05) | Wasser, gereinigt | 9698,5 |

Gegebenenfalls wird der pH-Wert durch Zugabe von 1N Salzsäure und/oder 1N Natronlauge korrigiert.

Die Ergebnisse zeigen, daß die Formulierung für keinen der beschriebenen Testorganismen einen geeigneten Nährboden zum Wachsen darstellt, sondern die Menge der Mikroorganismen gegenüber dem Inoculum deutlich abnimmt. Die Ergebnisse sind in Tabelle 1 zusammengefaßt, die das Wachstum von Mikroorganismen in isotonischen Formulierungen mit 0,05 Gew.% Xylometazolin zeigt. Unter der Rubrik Xylometazolin finden sich die Ergebnisse für die untersuchte Lösung ohne Silberfaden, in der Rubrik Xylometazolin + Silber finden sich die Ergebnisse für die Lösungen mit Silberfaden.

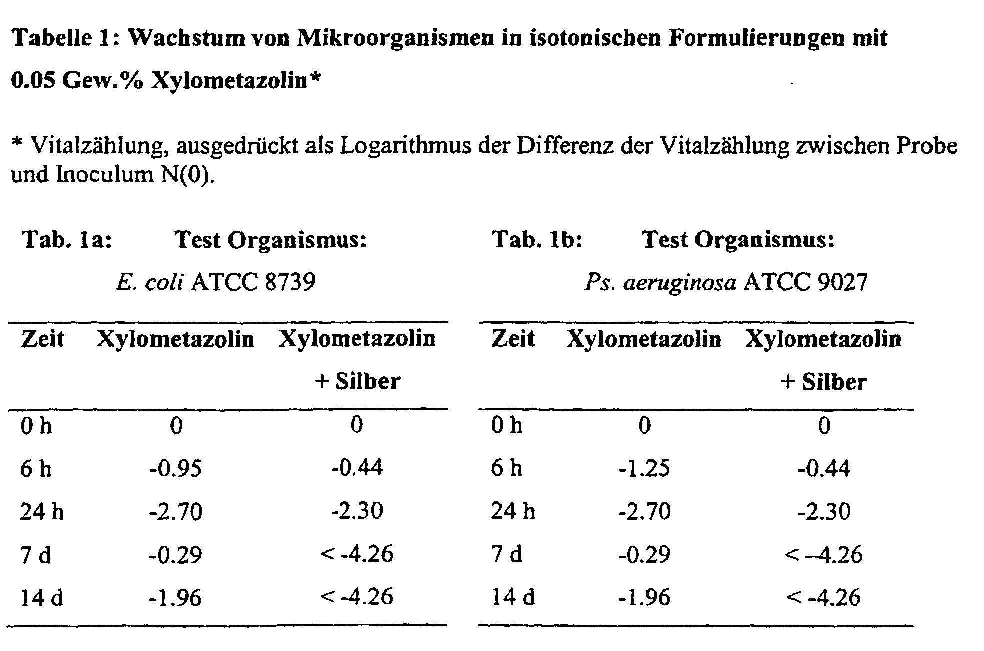

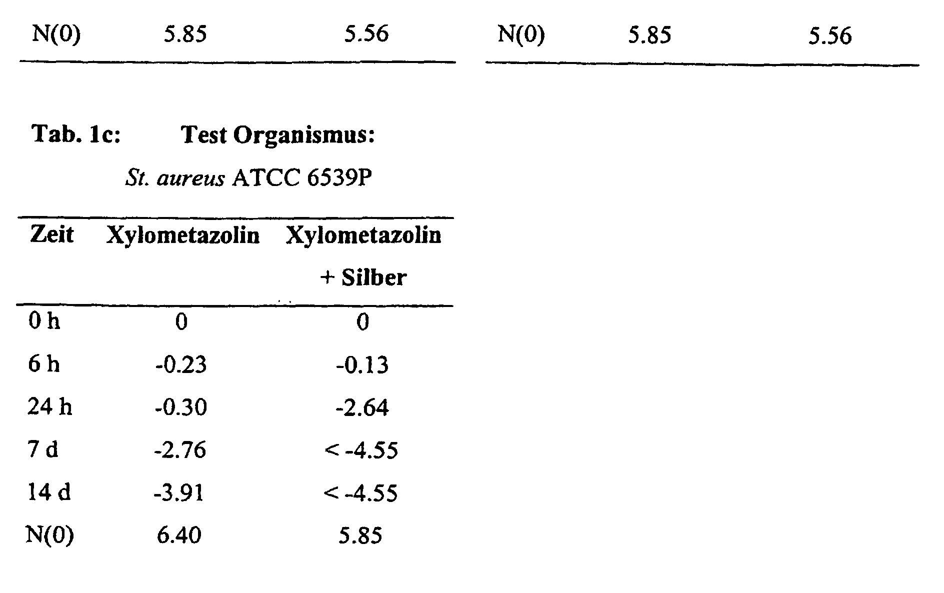

### Beispiel 2:

Untersuchung der folgenden 0,05 Gew.%igen Xylometazolinlösung mit einem pH-Wert von 6,0 auf biologische Stabilität:

| mg / 10 ml = 10150 mg | | |
|---|---|---|
| (01) | Xylometazolin Hydrochlorid | 10,0 |
| (02) | Sorbitol | 400,0 |
| (03) | Mononatriumdihydrogenphophat-Dihydrat | 39,5 |
| (04) | Dinatriummonohydrogenphophat-Dihydrat | 6,6 |
| (05) | Wasser, gereinigt | 9693,9 |

Gegebenenfalls wird der pH-Wert durch Zugabe von 1N Salzsäure und/oder 1N Natronlauge korrigiert.

Die Ergebnisse zeigen, daß die Formulierung für keinen der beschriebenen Testorganismen einen geeigneten Nährboden zum Wachsen darstellt, sondern die Menge der Mikroorganismen gegenüber dem Inoculum deutlich abnimmt. Die Ergebnisse sind in Tabelle 2 zusammengefaßt, die das Wachstum von Mikroorganismen in isotonischen Formulierungen mit 0,1 Gew.% Xylometazolin zeigt. Unter der Rubrik Xylometazolin finden sich die Ergebnisse für die untersuchte Lösung ohne Silberfaden, in der Rubrik Xylometazolin + Silber finden sich die Ergebnisse für die Lösungen mit Silberfaden.

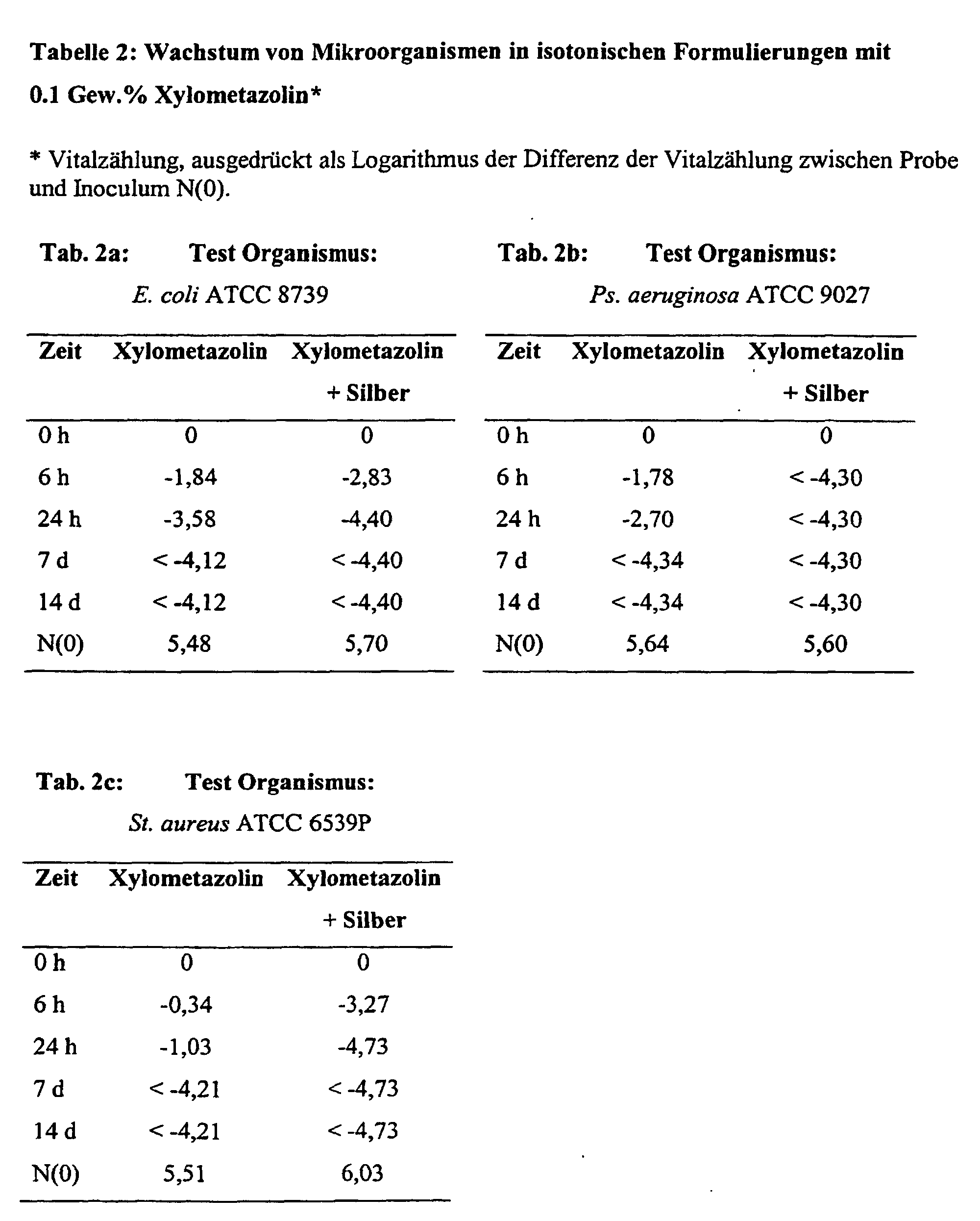

### Beispiel 3

| mg /10 ml =10146 mg | | |
|---|---|---|
| (01) | Xylometazolin Hydrochlorid | 5,0 |
| (02) | Sorbitol pulv. | 400,0 |
| (03) | Trometamol | 39,0 |
| (04) | Salzsäure 1N | 300,0 |
| (05) | Wasser, gereinigt | 9402,0 |

### Beispiel 4

| mg / 10 ml = 10146 mg | | |
|---|---|---|
| (01) | Xylometazolin Hydrochlorid | 5,0 |
| (02) | Sorbitol pulv. | 400,0 |
| (03) | Trometamol | 39,0 |
| (04) | Salzsäure 1N | 340,0 |
| (05) | Wasser, gereinigt | 9362,0 |

Anstelle des Xylometzolins kann auch Oxymetazolin verwendet werden.

Auch diese Formulierungen zeigen keine Anfälligkeit gegenüber mikrobiellem Wachstum. Bei den zugundeliegenden Versuchen wurden die Testformulierungen mit 10³ bis 10⁴ frisch gewachsenen Mikroorganismen inkubiert und bei 25°C 72 Stunden stehen gelassen. Die Ergebnisse werden durch Tabelle 3 und Tabelle 4 zusammengefasst.

**Tabelle 3:**

| **Wachstum von Mikroorganismen in Formulierungen mit 0.05 Gew.% Xylometazolin und Trometamol** | |
|---|---|
| Formulierung (mit jeweils 0,1 Gew.% Xylometazolin) | Sorbitol 4,0 Gew.% Trometamol Puffer pH 7,2 |
| Alcaligenes faecalis DSM 2576 | -- |
| Alcaligenes sp. DSM 6610 | -- |
| Flavobakterien sp. | -- |
| Sphingomonas paucimobilis DSM 1098 | -- |
| Pseudomonas aeruginosa ATCC 15442 | -- |
| Pseudomonas fluorescens DSM 6607 | -- |
| Pseudomonas fluorescens DSM 50106 | -- |
| Pseudomonas putida DSM 548 | -- |
| Pseudomonas putida DSM 291 | -- |
| Pseudomonas stutzeri DSM 6538 | - |
| Escherichia coli ATCC 8739 | - |
| Staphylococcus aureus ATCC 6538 | -- |
| Candida albicans ATCC 10231 | -- |
| Aspergillus niger ATCC 16404 | - |
| ++: starkes Wachstum (zwischen 1.5 und 3.0 log) | |
| +: moderates Wachstum (zwischen 0.5 und 1.5 log) | |
| 0: keine signifikante Veränderung | |
| -: moderate Abnahme (zwischen 0.5 und 1.5 log) | |
| --: starke Abnahme (zwischen 1.5 und 3.0 log) | |

**Tabelle 4:**

| **Wachstum von Mikroorganismen in Formulierungen mit 0.05 Gew.% Oxymetazolin und Trometamol** | | |
|---|---|---|
| Formulierung (mit jeweils 0,05 Gew.% Oxymetazolin) | Sorbitol 4,0 Gew.% Trometamol Puffer pH 7,0 | NaCl 0,9 Gew.% ungepuffert pH 7,0 |
| Alcaligenes faecalis DSM 2576 | - | 0 |
| Alcaligenes sp. DSM 6610 | 0 | -- |
| Flavobakterien sp. | -- | -- |
| Sphingomonas paucimobilis DSM 1098 | 0 | + |
| Pseudomonas aeruginosa ATCC 15442 | 0 | ++ |
| Pseudomonas fluorescens DSM 6607 | -- | ++ |
| Pseudomonas fluorescens DSM 50106 | 0 | ++ |
| Pseudomonas putida DSM 548 | -- | ++ |
| Pseudomonas putida DSM 291 | - | ++ |
| Pseudomonas stutzeri DSM 6538 | -- | - |
| Escherichia coli ATCC 8739 | -- | ++ |
| Staphylococcus aureus ATCC 6538 | -- | -- |
| Candida albicans ATCC 10231 | -- | 0 |
| Aspergillus niger ATCC 16404 | 0 | - |
| ++: starkes Wachstum (zwischen 1.5 und 3.0 log) | | |
| +: moderates Wachstum (zwischen 0.5 und 1.5 log) | | |
| 0: keine signifikante Veränderung | | |
| -: moderate Abnahme (zwischen 0.5 und 1.5 log) | | |
| --: starke Abnahme (zwischen 1.5 und 3.0 log) | | |

## Patentansprüche

1. Stabile isotonische Lösungsformulierung mit einem pH-Wert von 4,5- 7,5 bestehend aus
- Xylometazolinhydrochlorid und/oder Oxymetazolinhydrochlorid als Wirkstoff,
- einem pharmakologisch für die nasale Applikation verträglichen Lösungsmittel,
- einem Adjuvans aus der Gruppe Sorbitol und/oder Glycerol,
- einen Puffer aus der Gruppe der anorganischen pH-Puffer oder dem organischen Puffer Trometamol und
- gegebenenfalls Salzsäure und/oder Natronlauge,
- ggf. zum Einstellen der Isotonie notwendige Substanzen,
- gegbenenfalls einem oligodynamisch wirksamen Metall oder oligodynamisch wirksamen Metallionen in pharmazeutisch akzeptabler Menge,
wobei die Formulierung keine weiteren organischen Zusatzstoffe enthält.

2. Formulierung nach Anspruch 1, **dadurch gekennzeichnet, daß** der Puffer ein anorganischer Puffer ist.

3. Formulierung nach Anspruch 2, **dadurch gekennzeichnet, daß** die Lösung einen Natriumund/oder Kalium-Phosphatpuffer oder einen Natrium- und/oder Kalium-boratpuffer enthält.

4. Formulierung nach Anspruch 3, **dadurch gekennzeichnet, daß** die Lösung einen Mononatriumdihydrogen-dinatriummonohydrogen-Phosphatpuffer und/oder Monokaliumdihydrogen-dikaliummonohydrogen-Phosphatpuffer enthält.

5. Formulierung nach Anspruch 1, **dadurch gekennzeichnet, daß** der Puffer Trometamol ist.

6. Formulierung nach einem der Ansprüche 1 bis 5, **dadurch gekennzeichnet, daß** in der Lösung ein pH-Wert von 5,0-7,2 eingestellt ist.

7. Formulierung nach einem der Ansprüche 1 bis 4, **dadurch gekennzeichnet, daß** in der Lösung ein pH-Wert von 5,5-6,8, bevorzugt 5,8-6,0 eingestellt ist.

8. Formulierung nach Anspruch 5, **dadurch gekennzeichnet, daß** in der Lösung ein pH-Wert von 6,1-6,3 eingestellt ist.

9. Formulierung nach einem der Ansprüche 1 bis 8, **dadurch gekennzeichnet, daß** der Wirkstoff in einer Konzentration zwischen 0,01 und 1,0 Gew.%, bevorzugt zwischen 0,01 und 0,5 Gew.% und ganz besonders bevorzugt zwischen 0,05 und 0,1 Gew.% vorliegt.

10. Formulierung nach einem der Ansprüche 1 bis 9, **dadurch gekennzeichnet, daß** das Lösungsmittel Wasser ist.

11. Formulierung nach einem der Ansprüche 1 bis 9, **dadurch gekennzeichnet, daß** das Lösungsmittel ein Ethanol-Wasser-Gemisch ist.

12. Formulierung nach einem der Ansprüche 1 bis 11, **dadurch gekennzeichnet, daß** der Anteil des Adjuvans an der Lösung 1 bis 10 Gew.%, bevorzugt 2 bis 6 Gew.% beträgt.

13. Formulierung nach Anspruch 12, **dadurch gekennzeichnet, daß** das Adjuvans 3,5 bis 4,5 Gew.%, bevorzugt 4,0 Gew.%, Sorbitol ist.

14. Formulierung nach Anspruch 12, **dadurch gekennzeichnet, daß** das Adjuvans 2,0 bis 2,8 Gew.%, bevorzugt 2,4 Gew.%, Glycerol ist.

15. Formulierung nach einem der Ansprüche 1 bis 14, **dadurch gekennzeichnet, daß** die Formulierung kein oligodynamisch wirkendes Metall oder oligodynamisch wirkende Metallionen enthält.

16. Formulierung nach einem der Ansprüche 1 bis 14, **dadurch gekennzeichnet, daß** die Formulierung ein oligodynamisch wirkendes Metall oder oligodynamisch wirkende Metallionen enthält.

17. Formulierung nach Anspruch 16, **dadurch gekennzeichnet, daß** das oligodynamisch wirkende Metall oder die oligodynamisch wirkende Metallionen Silber ist oder Silberionen sind.

18. Formulierung nach einem der Ansprüche 1 bis 17, **dadurch gekennzeichnet, daß** die Formulierung nur Xylometazolinhydrochlorid als Wirkstoff enthält.

19. Formulierung nach einem der Ansprüche 1 bis 17, **dadurch gekennzeichnet, daß** die Formulierung nur Oxymetazolinhydrochlorid als Wirkstoff enthält.

20. Verwendung einer Formulierung nach Anspruch 1 bis 19 zusammen mit einem Inhalator mit silberhaltigen Elementen im Bereich zwischen Wirkstoffreservoir und Sprühkopf.

21. Verwendung der Formulierung nach einem der Ansprüche 1 bis 19 zur Herstellung eines Rhinologikums.

## Claims

1. Stable isotonic formulation of a solution having a pH of 4.5 - 7.5 consisting of
- xylometazoline hydrochloride and/or oxymetazoline hydrochloride as active substance,
- a solvent which is pharmacologically acceptable for nasal administration,
- an adjuvant selected from among sorbitol and/or glycerol,
- a buffer selected from among the inorganic pH buffers or the organic buffer Trometamol and
- optionally hydrochloric acid and/or sodium hydroxide solution,
- optionally substances needed to render the solution isotonic,
- optionally an oligodynamically active metal or oligodynamically active metal ions in pharmaceutically acceptable amounts,
the formulation containing no other organic additives.

2. Formulation according to claim 1, **characterised in that** the buffer is an inorganic buffer.

3. Formulation according to claim 2, **characterised in that** the solution contains a sodium and/or potassium phosphate buffer or a sodium and/or potassium borate buffer.

4. Formulation according to claim 3, **characterised in that** the solution contains a monosodium dihydrogen-disodium monohydrogen phosphate buffer and/or monopotassium dihydrogen-dipotassium monohydrogen phosphate buffer.

5. Formulation according to claim 1, **characterised in that** the buffer is Trometamol.

6. Formulation according to one of claims 1 to 5, **characterised in that** the solution is adjusted to a pH of 5.0 to 7.2.

7. Formulation according to one of claims 1 to 4, **characterised in that** the solution is adjusted to a pH of 5.5 to 6.8, preferably 5.8 to 6.0.

8. Formulation according to claim 5, **characterised in that** the solution is adjusted to a pH of 6.1 to 6.3.

9. Formulation according to one of claims 1 to 8, **characterised in that** the active substance is present in a concentration of between 0.01 and 1.0% by weight, preferably between 0.01 and 0.5% by weight and most preferably between 0.05 and 0.1% by weight.

10. Formulation according to one of claims 1 to 9, **characterised in that** the solvent is water.

11. Formulation according to one of claims 1 to 9, **characterised in that** the solvent is a mixture of ethanol and water.

12. Formulation according to one of claims 1 to 11, **characterised in that** the proportion of adjuvant in the solution is 1 to 10% by weight, preferably 2 to 6% by weight.

13. Formulation according to claim 12, **characterised in that** the adjuvant is 3.5 to 4.5% by weight, preferably 4.0% by weight, of sorbitol.

14. Formulation according to claim 12, **characterised in that** the adjuvant is 2.0 to 2.8% by weight, preferably 2.4% by weight, of glycerol.

15. Formulation according to one of claims 1 to 14, **characterised in that** the formulation contains no oligodynamically active metal or oligodynamically active metal ions.

16. Formulation according to one of claims 1 to 14, **characterised in that** the formulation contains an oligodynamically active metal or oligodynamically active metal ions.

17. Formulation according to claim 16, **characterised in that** the oligodynamically active metal or oligodynamically active metal ions is silver or are silver ions.

18. Formulation according to one of claims 1 to 17, **characterised in that** the formulation contains only xylometazoline hydrochloride as active substance.

19. Formulation according to one of claims 1 to 17, **characterised in that** the formulation contains only oxymetazoline hydrochloride as active substance.

20. Use of a formulation according to claims 1 to 19 together with an inhaler having silver-containing elements in the region between the active substance reservoir and the sprayhead.

21. Use of a formulation according to one of claims 1 to 19 for the preparation of a rhinological agent.

## Revendications

1. Formulation de solution isotonique stable avec une valeur du pH de 4,5-7,5, consistant en
- Chlorhydrate de xylométazoline et/ou chlorhydrate d'oxymétazoline en tant que principe actif,
- un solvant compatible d'un point de vue pharmacologique pour l'administration nasale,
- un adjuvant du groupe sorbitol et/ou glycérol,
- un tampon du groupe des tampons pH inorganique ou du tampon organique trométamol et
- éventuellement, l'acide chlorhydrique et/ou une lessive de soude,
- des substances éventuellement nécessaires pour l'ajustement de l'isotonie,
- éventuellement, un métal oligodynamiquement actif ou des ions métalliques oligodynamiquement actifs dans une quantité acceptable d'un point de vue pharmaceutique,
la formulation ne contenant pas d'autres additifs organiques.

2. Formulation selon la revendication 1, **caractérisée en ce que** le tampon est un tampon inorganique.

3. Formulation selon la revendication 2, **caractérisée en ce que** la solution contient un tampon de phosphate de sodium et/ou de potassium ou un tampon de borate de sodium et/ou de potassium.

4. Formulation selon la revendication 3, **caractérisée en ce que** la solution contient un tampon de phosphate monosodium dihydrogène-disodium monohydrogène et/ou un tampon de phosphate monopotassium dihydrogène-dipotassium monohydrogène.

5. Formulation selon la revendication 1, **caractérisée en ce que** le tampon est un trométamol.

6. Formulation selon l'une des revendications 1 à 5, **caractérisée en ce que** l'on ajuste le pH à une valeur de 5,0-7,2 dans la solution.

7. Formulation selon l'une des revendications 1 à 4, **caractérisée en ce que** l'on ajuste le pH à une valeur de 5,5-6,8, de préférence 5,8-6,0, dans la solution.

8. Formulation selon la revendication 5, **caractérisée en ce que** l'on ajuste le pH à une valeur de 6,1-6,3 dans la solution.

9. Formulation selon l'une des revendications 1 à 8, **caractérisée en ce que** le principe actif est présent dans une concentration entre 0,01 et 1,0 % en poids, de préférence entre 0,01 et 0,5 % en poids, et de façon tout particulièrement préférée entre 0,05 et 0,1 % en poids.

10. Formulation selon l'une des revendications 1 à 9, **caractérisée en ce que** le solvant est de l'eau.

11. Formulation selon l'une des revendications 1 à 9, **caractérisée en ce que** le solvant est un mélange éthanol-eau.

12. Formulation selon l'une des revendications 1 à 11, **caractérisée en ce que** la quantité de l'adjuvant dans la solution est de 1 à 10 % en poids, de façon préférée de 2 à 6 % en poids.

13. Formulation selon la revendication 12, **caractérisée en ce que** l'adjuvant est le sorbitol de 3,5 à 4,5 % en poids, de façon préférée à 4,0 % en poids.

14. Formulation selon la revendication 12, **caractérisée en ce que** l'adjuvant est le glycérol de 2,0 à 2,8 % en poids, de façon préférée à 2,4 % en poids.

15. Formulation selon l'une des revendications 1 à 14, **caractérisée en ce que** la formulation ne contient aucun métal actif oligodynamiquement ou ions métalliques actifs oligodynamiquement.

16. Formulation selon l'une des revendications 1 à 14, **caractérisée en ce que** la formulation contient un métal actif oligodynamiquement ou des ions métalliques actifs oligodynamiquement.

17. Formulation selon la revendication 16, **caractérisée en ce que** le métal actif oligodynamiquement ou les ions métalliques actifs oligodynamiquement sont l'argent ou des ions argent..

18. Formulation selon l'une des revendications 1 à 17, **caractérisée en ce que** la formulation ne contient que du chlorhydrate de xylométazoline en tant que principe actif.

19. Formulation selon l'une des revendications 1 à 17, **caractérisée en ce que** la formulation ne contient que du chlorhydrate d'oxymétazoline en tant que principe actif.

20. Utilisation d'une formulation selon la revendication 1 à 19 conjointement avec un inhalateur, avec des éléments contenant de l'argent dans la zone située entre le réservoir du principe actif et la tête de pulvérisation.

21. Utilisation de la formulation selon l'une des revendications 1 à 19 pour la préparation d'une spécialité rhinologique.
